# EUROPEAN PATENT APPLICATION

(11) **EP 4 527 286 A1**
(43) Date of publication of application: **26.03.2025**
(21) Application number: 24200065.1
(22) Date of filing: 12.09.2024
(51) Int. Cl.: A61B 3/12, A61N 5/06, F21K 9/61, F21V 8/00, G02B 27/09, F21Y 113/13

(54) **LIGHT SOURCE, SYSTEM OF LIGHT SOURCES, AND DEVICE FOR RETINAL DIAGNOSTIC AND/OR THERAPY**

(30) Priority: 21.09.2023 CH 10382023
(71) Applicant: OculoWise, 1950 Sion (CH)
(72) Inventor: Geiser, Martial, 1950 Sion (CH); Truffer, Frederic, 3928 Randa (CH)
(74) Representative: P&TS SA (AG, Ltd.)

(57) **Abstract**

A light source (1) for emitting polychromatic spectral homogeneous light in a spectral band, the light source (1) comprising: a plurality of light-emitting members (2), wherein each configured to emit light in a specific single spectral band and each coupled to a light guide (6) forming a bundle, an optical mixer that homogenises the light emitted by emitting members (2). The source preferably includes a spectra-photometer subsystem that can be used achieve a desired spectral intensity and may be integrated into a medial device for retinal diagnostic and/or therapy (30).

## Description

### Technical domain

The present invention concerns, in embodiments, a light source for emitting polychromatic spectral homogeneous light in a spectral band, as well as a system of light sources, and an ophthalmic retina diagnostic and/or a retina therapy device.

### Related art

Optical systems are widely used in clinical and research ophthalmology. Conventional lighting systems are poorly versatile and flexible. The lack of fine tunability of critical parameters: spectral characteristics, intensity profile, and field of illumination will miss to realize several opportunities in specific imaging modalities and defined ophthalmic procedures.

Modern electro-optical systems allow for the acquisition of highly structured images in complex - interdependent scenarios. The increased understanding and the observed clinical data focused on light-tissue interactions enable the advent of new therapeutic protocols. These capabilities drive and rise the need to further optimise sample illumination-stimulation. The ability to precisely control the illumination parameters, including the spectral output, intensity, and spatial distribution of light, is critical.

In the field of ophthalmology, the ability to adjust spectroscopy and intensity of the light projected into the patient's eye allows for optimal fundus illumination without overwhelming sensitive tissues or causing discomfort to the patient. Capturing images or examining the eye with consistent lighting conditions is crucial to enhance diagnosis capabilities or assess and image eye's structures with higher accuracy.

Non-uniform spectral illumination fields will introduce brightness and colour variations, interfering with image interpretation and diagnosis. In the same manner, in light-tissue therapeutics, spectral inhomogeneities will results in un-even tissue stimulation. Finely-tuned and controlled light intensity protocols ensures the acquisition of crispy and detailed images without compromising patient safety. In addition to the imaging application, a therapeutic light source guaranteeing uniform intensity distribution will ensures an even brightness across the entire field.

US2010219758A1 discloses a lighting device with a plurality of controllable light-emitting diodes (LEDs). The invention aims to provide a lighting solution that offers enhanced control and flexibility in adjusting the intensity, colour, and distribution of light emitted by the LEDs.

US2011292343A1 discloses a spectrally-adjustable ophthalmic illumination system with discrete light sources. The invention aims to provide an ophthalmic illumination system that offers adjustable spectral output to enhance various ophthalmic procedures and examinations.

However, both solutions known from the prior art are complex, power-demanding, and expensive. The proposed innovative lighting device provides a finely tuneable spectral output with a uniform field of excitation/view in a simple and cost-effective solution.

### Short disclosure of the invention

An object of the present invention is the provision of a light source, a system of light sources, and a device for retinal diagnostic and/or therapy that overcomes the shortcomings and limitations of the state of the art. The light source, the system and the device are small in size, comprise only a few optical, mechanical and electrical components and are, therefore, less complex.

A further object is the provision of a light source, a system of light sources, and a device for retinal diagnostic and/or therapy that can output light that exhibits both spectral evenness across the entire spectrum of wavelengths and spatial evenness in the distribution of light intensity.

A secondary object is the provision of a light source, a system of light sources, and a device for retinal diagnostic and/or therapy that can output light with the properties mentioned in the paragraph before, and can be designed to emit light in two operational modes. In a first mode, it can output light with a relatively low radiant flux. However, it can also be configured to switch to a second operational mode, where it outputs light with a much higher radiant flux. A combination of the two modes mentioned can also be possible.

According to a first aspect of the present invention, a light source for emitting polychromatic spectral homogeneous light in a spectral band involving the features recited in claim 1 is disclosed.

The light source comprising:
- a plurality of light-emitting members, wherein each light-emitting member of the plurality of light-emitting members is configured to emit light in a specific single spectral band, the said single spectral band being characterised by a central wavelength and a spectral bandwidth; and
- a number of light guides corresponding to the number of light-emitting members, each light guide being optically connected at one end to a corresponding light-emitting member, the plurality of light guides being arranged at another end to form a light guide bundle; and
- an optical light mixing member, the optical light mixing member is configured with an optical input and an optical output, wherein the optical input is optically connected to the light guide bundle, and wherein the optical light mixing member is configured to mix and homogenise spectrally the light emitted by the plurality of light-emitting members, wherein the optical output is provided to output the mixed and homogenised light for emitting the polychromatic spectral homogeneous light.

The light emitted by the light source at the optical output can have the same radiant flux and same spectrum when looking at each point at the optical output, thereby providing light with a spectral evenness across the entire spectrum of wavelengths and spatial evenness in the distribution of the light intensity. This is, among other factors, enabled by the optical light mixing member, which collects the light emitted by the light-emitting members at its optical input, and subsequently blends the light from specific, single spectral bands while aligning the radiant flux at the same time.

The light-emitting members can be configured as discrete members, meaning that they can be separate and distinct from each other. Each light-emitting member is provided to emit light in a specific single spectral band, whereby specific can relate to a distinct single spectral band. The spectral band selected can be mutually different amongst the light-emitting members. The light-emitting member can be configured as monochromatic light-emitting diodes (LEDs), whereby it is known by a skilled person in the art that monochromatic LEDs emit light containing different wavelengths from a narrow spectral band. In fact, they can emit polychromatic light, even if they are called monochromatic. The light-emitting members can also be provided as Gas Discharge or Atomic Emission Spectral Lamps, lasers (including laser diodes, or tuneable lasers), etc. The alternatives provided are not exhaustive.

The phrase "optically connected" can refer to establishing a connection or link between two or more optical devices or components. The optical input can refer to a port or interface on an optical component that is specifically provided to receive light, such as in the form of a beam, whereby optical output refers to the contrary, namely a port or interface on an optical component that is specifically provided to transmit the said light received at the optical input. Optical inputs and outputs are sometimes referred to as optical interfaces. The light guides can be provided as optical fibres, planar waveguides, graded-index fibres, holey fibres, glass or plastic rods, etc, whereby the list is not exhaustive.

The light source can be used in various applications. For example, the optical output can be directed at an object to be illuminated, as in the field of light microscopy. For this purpose, the optical output of the light source can be integrated into the light microscope, whereby the optical output emits light in the direction of the stage, i.e. onto the specimen to be illuminated. The optical output of the light source can also be used by external components or devices, such as used for light guide illumination applications, in which the light guide of an external device can be optically coupled to the optical output of the light source. The example applications mentioned before a just a tiny subset of applications in which the light source can be used,

In a first embodiment of the first aspect, the spectral band emitted by the light source can contain wavelengths spanning from 200 nm to 1300 nm, more preferably from 380 nm to 780 nm or to 810 nm, or to 830. The latter range can correspond to the visible range of a human eye. The shortest and longest wavelengths can define the limits of the spectrum that the light source can emit.

In a second embodiment of the first aspect, the central wavelength of the plurality of light-emitting members can be selected to be approximately equally spaced within the spectral band. This circumstance can enable or at least support the spectral homogeneity of the light that the light source can emit. In addition, and in the field of ophthalmology, this selection (=approximately equally spaced) can be useful for fine-tuning the stimulation of blue and ipRGC photoreceptors. In particular, three types of photoreceptor cells exist in the human retina: rods (sensitive to all visible wavelengths, peak at around 498 nm), cones ( three color-sensitive, peak at around 420 nm, 533 nm and 564 nm), and photosensitive retinal ganglion cells (pRGC) containing melanopsin, sensitive to short-wavelength blue light. Melanopsin's spectral absorption and human nocturnal melatonin suppression sensitivity peak in the blue spectrum at 480 nm, respectively.

In a third embodiment of the first aspect, some of the specific single spectral bands of the plurality of light-emitting members can be selected non-overlapping. The light source can thereby emit light with distinct spectral bands characterised by their bandwidth and centred wavelength. This can mean, in return, that other specific single spectral bands can be selected to overlap.

In a further embodiment of the first aspect, the light-emitting members can be configured as LEDs. In the present embodiment, preferably each light-emitting member is configured as an LED. Preferably surface-mount LEDs are used, each emitting light in a specific predetermined spectral band. The selected LEDs' bandwidth is preferably smaller than 60 nm, preferably smaller than 40 nm most preferably smaller than or equal to 30 nm, determined at full width at half maximum (FWHM).

In a different embodiment of the first aspect, the LEDs of the light source can be configured to emit light in at least six mutually different spectral bands, wherein said six mutually different spectral bands can comprise or can be centred at the following six wavelengths: 420 nm, 450 nm, 470 nm, 520 nm, 590 nm, and 630 nm. The centred wavelength can refer to the wavelength determined at the maximum intensity. In the following, the number of light-emitting members or LEDs accounts for six or seven, respectively. Of course, the light source can also be configurated with any number smaller or greater than the six, or seven light-emitting members or LEDs mentioned. The same applies to the number of spectral bands, centred wavelengths and light guides mentioned, as they correspond to the number of light-emitting members or LEDs selected. The number and the optical properties of the light-emitting members or LEDs might be designed or selected in response to the application in which the light source is used. Each LED can be configured with a maximum power rating of smaller than 1 W, more preferably smaller than 500 mW, most preferably smaller than or equal to 200 mW. For standard illumination applications, the LED can be selected with the highest maximum power rating mentioned, whereas for ophthalmologic applications, a maximum power of 200 mW or lower is preferred not to overstimulate or damage the retinal cells.

In another embodiment of the first aspect, comprising a light-emitting member in excess of the number of a plurality of light-emitting members, wherein said excess light-emitting member is configured to emit light in a mutually different spectral band, wherein the mutually different spectral band comprises or is centred at a wavelength between 790 nm and 830 nm, or 800 nm and 820 nm or at 810 nm. The term "in excess" can refer to the fact that the excess light-emitting member can hold other electrical or optical properties compared to the plurality of light-emitting member mentioned before. The excess light-emitting member can also be configured as LED emitting light in the spectral band or with the wavelength as defined in the present embodiment. When configured as LED, the excess light-emitting member can also be referred to as the seventh excess light-emitting member or seventh LED. The wavelength mentioned can be useful to stimulate retinal cells when the light source is used for ophthalmologic applications. Light emitted within the selected wavelength can provide the advantage that the light deeply penetrates the tissue of the eye. The excess light-emitting member can be selected or can be controlled to output a higher radiant flux than the remaining six light-emitting members mentioned before. Therefore the excess light-emitting member or excess LED can provide light for treatments, and the six remaining LEDs can provide light for the stimulating or illumination of the fundus. The maximum power rating of the excess light-emitting member or excess LED is higher than 200 mW, more preferably higher than 500 mW, most preferably higher than or equal to 1 W.

In a further embodiment of the first aspect, the light source can comprise one light guide in excess of a number of light guides corresponding to the number of light-emitting members, wherein the one light guide can be at one end optically connected to a secondary light source external to the light source, and can be at another end formed as part of the light guide bundle, wherein the secondary light source can be configured to emit light at a wavelength centred between 790 nm and 830 nm, or 800 nm and 820 nm or at a wavelength of 810 nm. Instead of providing a dedicated light-emitting member for the treatment, light within the spectral band or with the wavelength as defined in the present embodiment can be decoupled from an external light source into the one light guide. The secondary light source can be configured as a laser light source providing light with a higher radiant flux than the number of light-emitting members disclosed before. This secondary light source, in combination with the light source according to the invention, can also be used for ophthalmologic applications, offering the advantages outlined before.

In a different embodiment of the first aspect, each light guide can be configured as a multi-mode optical fibre, preferably with a core diameter of 1 mm and a length between 50 mm and 120 mm. Even though multi-mode optical fibre can offer low optical losses, the length should be as short as possible to avoid bending. Each light guide's numerical aperture can be between 0.2 and 0.4.

In another embodiment of the first aspect, the light mixing member can be configured as a straight hexagonal glass light pipe, preferably configured with a long diagonal of 3 mm and a length of at least 40 mm. The numerical aperture of the light mixing member in the present embodiment can account for approximately 1. The light emitted by the light-emitting members, regardless of the wavelength, can be sufficiently well mixed by internal reflections of the straight hexagonal glass light pipe. The inventor found an optimum of diameter to length.

In a further embodiment of the first aspect, the light mixing member can be configured as an optical fibre with a constant diameter, preferably configured with a diameter of 3 mm and a length of at least 40 mm. The diameter can be constant along the entire elongation of the optical fibre. Optical fibre can be made of optical glass or plastic material and can also be provided with a numerical aperture of close to 1. However, the numerical aperture can be smaller than 1 if it matches the subsequent system.

In another embodiment of the first aspect, the light mixing member can be configured as a tapered optical fibre, wherein a non-tapered end can correspond to the optical input of the light mixing member, and preferably the non-tapered end can be configured with a diameter of 3 mm and a tapered end can be configured with a diameter of 1 mm. The optical fibre can be tapered along the elongation of the said fibre. The numerical input aperture can also be close to 1, whereby the numerical output aperture can account for approximately 0.33. In fact, the numerical aperture of the optical input of the light mixing member must be greater than the numerical aperture of each light guide. The tapered optical fibre can be made of optical glass or plastic material, whereby optical glass offers lower optical losses.

In a further embodiment of the first aspect, the light guide bundle can be formed in a shape corresponding to a shape of the light mixing member, and wherein a surface area of the said shape can be equal to a surface area of the optical input of the optical light mixing member. Consequently, the shape of the light guide bundle approximates the shape of the optical input of the light mixing member, which improves the optical coupling of the light outputted by the light guide bundle. Optical losses at the interface between the optical light mixing member input and the light guide outputs. The same shape also facilitates mechanical joining.

In a different embodiment of the first aspect, the light source can further comprise a printed circuit board (PCB), wherein the light-emitting members can be placed on a first side of the PCB, the light source further comprising one or a plurality of Peltier elements placed on a side opposite to the first side, wherein the Peltier elements can be arranged to influence a temperature of the PCB. A Peltier element can be arranged directly below an LED so that there can be good thermal contact between a Peltier element and an LED, through the PCB, whereby the PCB separates the LEDs from the Peltier elements. The Peltier elements can be used to cool the LEDs. In cold environments, the Peltier elements can be used to heat the PCB and, finally the LEDs.

In a different embodiment of the first aspect, the light source can further comprise a heatsink arranged in thermal contact with the Peltier elements, and the heatsink can be configured to transfer heat generated by the Peltier elements to the environment. The Peltier elements can be in thermal contact with the heatsink.

In another embodiment of the first aspect, the light source can further comprise an absorptive filter for excitation of the light emitted by the plurality of light-emitting members, wherein the absorptive filter can be arranged in optical connection with the optical input of the optical light mixing member and the light guide bundle. The absorptive filter can be configured as a grey filter.

In further embodiment of the first aspect, the light source can further comprise a LED driver circuit operably coupled to the plurality of LEDs, wherein the LED driver circuit can be configured to selectively drive each LED from the plurality of LEDs. Each LED can be individually supplied with a related supply current, whereby the supply current's amperage can be controlled to adjust the radiant flux emitted.

In a different embodiment of the first aspect, the light source can further comprise a control unit operably coupled to the LED driver circuit and configured to control a radiant flux of each LED from the plurality of LEDs by selectively setting a driving current supplied by the LED driver circuit to the LEDs. The driving current can also refer to as the supply current. The supply current can be a direct (DC) current, or more preferably a pulsed current, whereby the amperage (mean value) is controlled using pulse-width modulation techniques.

In a further embodiment of the first aspect, the control unit can be configured to control the radiant flux of each LED to a predetermined value for generating a specific spectral power distribution within the spectral band alongside of the emitted specific single spectral band for silent substitution stimulation of a retina comprised in an eye external to the light source. In fact, the human pupillary light response is driven by various photoreceptors in the eye, including cones, rods, and intrinsically photosensitive retinal ganglion cells (ipRGCs) expressing melanopsin. These photoreceptors have distinct but overlapping spectral tuning. Silent substitution is used as a method in ophthalmology that utilises pairs of lights ("metamers") to selectively stimulate one class of photoreceptors while keeping the activation of all others constant (= silent). The control unit can expressly be foreseen and configured to control the light outputted (e.g. spectrally) to enable silent substitution stimulation using the light source according to the invention.

There are many challenges to silent substation stimuli, where some of them arising from the light source used. The imperfect human eye, in cases of spatially extended light sources with external light, may cause undesired stimulation of unadopted photoreceptors. Furthermore, the light source may exhibit instability over time, leading to changes in spectral output between operations or throughout sessions. All of the mentioned challenges are widely mitigated by the light source according to the invention since light with a spectral evenness across the entire spectrum of wavelengths and spatial evenness in the distribution of the light intensity is emitted.

In a different embodiment of the first aspect, the control unit can be configured to sequentially drive the LEDs to traverse the available spectral band, preferably by starting with the LED configured with the shortest or the longest centred wavelength.

In another embodiment of the first aspect, the light source can further comprise at least one temperature sensor operably coupled to the control unit and, wherein the at least one temperature sensor can be placed close to a LED, wherein the control unit can be configured to control an operation of the Peltier elements or an operation of the LED driver circuit in dependency of a temperature sensor signal provided by the at least one temperature sensor. The power supplied to the Peltier elements can be adjusted by the control unit or a related control instance so that the temperature of the PCB and the LEDs can be controlled in dependency on the measured temperature determined. Alternatively, the operation of the LED driver circuit may be terminated if the temperature measured exceeds a predetermined threshold. Terminating the operation may prevent damage to the LEDs.

Even though the individual embodiments of the first aspect cover different aspects of the invention, some or all embodiments can be combined when it is useful and feasible from a technical standpoint.

In a second aspect of the invention, a system of light sources is disclosed. The system comprises a plurality of light sources configured according to the first aspect (including any embodiment or a combination thereof);
- a master control unit operably coupled to each control unit of the plurality of light sources, wherein the master control unit is configured to control the radiant flux of each light-emitting member comprised in the plurality of light sources as a function of time by providing control signals to each control unit.

The system of light sources can form a single powerful compact light source, where the radiant flux of each light-emitting member comprised in the single powerful light can be controlled as a function of time. In particular, when a high light output in terms of high radiant power is required, multiple light sources of the first aspect can be combined into a system of light sources, thereby forming said single powerful light source. This can be useful when large areas need to be illuminated, or high radiant power is required such as for the treatment of eyes. However, the operation of the system's light sources must be carefully controlled by the master control unit, preferably in accordance with a predetermined protocol, such that illumination targets (radiant flux, spectral band emitted, etc.) are met.

In a first embodiment of the second aspect, the system can comprise a light collecting device with a collector input and a collector output, wherein each optical output of the plurality of light sources can be optically connected to the collector input, wherein the collector output can be configured to output light with a radiant flux corresponding to approximately a sum of radiant fluxes received at the collector input. The light collecting device can be configured to combine the light emitted by the optical output of each light source so that an increased radiant flux can be emitted at the system optical output corresponding to the collector output and/or light with an extended electromagnetic spectrum can be outputted, suppose that each light source is configured with a set of light-emitting members emitting light in a mutually different spectral band or with a mutually different centred wavelength. The control of each light source employing the master control unit can be required to stipulate the synergetic effect as outlined.

In a second embodiment of the second aspect, the light collecting device can be configured as a prism or a lens.

In a third aspect of the invention, a device for retinal diagnostic and/or therapy is disclosed. The device comprises:
- at least one light source configured according to the first aspect (including any embodiment or a combination thereof);
- a lens optically connected to the optical output of the at least one light source, wherein the lens is configured to collect the polychromatic spectral homogeneous light outputted by the light source and is further configured to focus the light collected onto a retina of an eye external to the device.

When the light source is configured with the six light-emitting members as mentioned, the light source can be used to illuminate the fundus of the eye. When the light source is configured with a seventh light emitting member (and/or a seventh light guide), the light source can be used for the treatment purposes, such as the stimulation of the retina. The lens used for this purpose might need to be selected accordingly. Dependent on the application, the optical setup can be more complex, whereby a plurality of different lenses might be used. Even then, the device benefits from the configuration, optical properties, and advantages of the light source.

In a first embodiment of the third aspect, the device can further be configured to emit two different fields of light to the eye, comprising a first and a second light source according to the first aspect (including any embodiment or a combination thereof), each optical output is optically connected to the lens, wherein a first field of light can be output by the first light source and a second field of light can be output by the second light source. The two different fields of light can be collected and combined into a common light field by the lens mentioned before.

In a second embodiment of the third aspect, the device can further comprise a refracting or reflecting element arranged in optical connection with the optical outputs of the first and the second light source, wherein said element can be configured to optically align the polychromatic spectral homogeneous light emitted by each light source. The refractive or reflective element can align the two field of light output by the optical outputs. The optical outputs can be distanced from each other. Using the refracting or reflecting element can be useful to specifically shape the common field of light, such as to output a coaxially aligned, partly or non-overlapping, partly cancelled, etc. field of light. The primary objective can therefore be, to generate at least two distinct fields presented simultaneously as for example left/one or bottom/up or central/external, which can be projected onto the fundus.

In a third embodiment of the third aspect, the said element can be optically arranged between each optical output of the light sources and the lens. This can be useful if the outputted combined field, are shaped by the said element and the combined field is focused on the fundus of the eye.

In a further embodiment of the third aspect, the refracting element can be configured as a prism, or the reflecting element can be configured as a mirror.

Even though the individual embodiments of the third aspect cover different aspects of the invention, some or all embodiments can be combined when it is useful and feasible from a technical standpoint.

The visible spectrum is often divided into parts named each after a different perceived colour. In the context of the present disclosure, the follow parts may be referred to: violet when λ≤440 nm, blue when 440 nm<λ≤490 nm, green when 490 nm<λ≤560 nm, yellow when 560 nm<λ≤590 nm, orange when 590 nm<λ≤610 nm and red when λ>610 nm. A blue/violet part is defined as the union of the violet and blue parts, corresponding to λ≤490 nm.

### Short description of the drawings

Exemplar embodiments of the invention are disclosed in the description and illustrated by the drawings in which:
Fig.1a schematically illustrates an example of the light source according to the invention.
Fig. 1b shows the output section of the light source of Fig. 1a in a detailed view.
Fig. 2 schematically illustrates variants of the optical light mixing members according to the invention.
Fig. 3a-3c shows variants of a retinal diagnostic and/or therapy device according to the invention in a simplified fashion.
Fig. 4 schematically illustrates a system of light sources, each being configured as shown in Fig. 1a, according to the invention.

### Examples of embodiments of the present invention

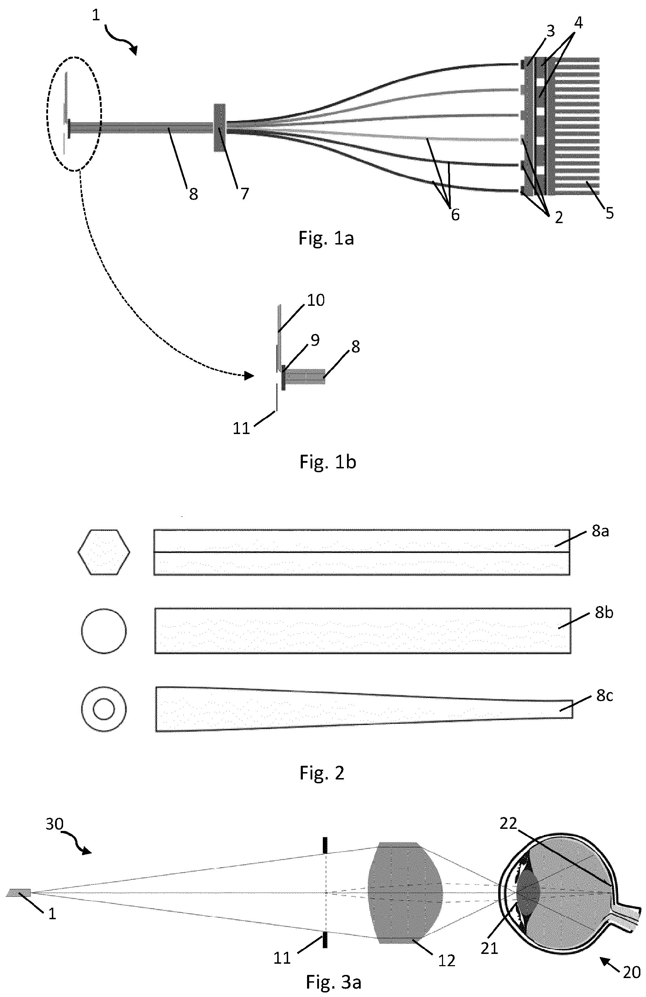

With reference to Fig. 1a, an example of the light source 1 according to the invention is schematically illustrated. A plurality of LEDs 2 are placed on a common PCB 3. It can be noticed in the figure that the PCB 3 holds a total number of six LEDs 2. Even though the LEDs 2 appear to be arranged one below the other, they can, for example, be arranged in any other geometry, such as in a circle. Furthermore, a seventh LED could be placed in the centre, and the six LEDs 2 encircle the seventh LED.

The six LEDs were selected to emit light (when powered) in six spectral bands, whereby each spectral band is characterised by a central wavelength (e.g. 500 nm) and a corresponding spectral bandwidth (e.g. 30nm, FWHM). The resulting electromagnetic spectrum that can be emitted, when all LEDs 2 are powered, contains wavelengths spanning from about 380 nm to approximately 750 nm. The LEDs 2 were selected such that they emit light with a high spectral homogeneity, meaning that the centred wavelength of the LEDs 2 is chosen so that all centred wavelengths are approximately equally spaced to each other, ranging in the electromagnetic spectrum mentioned before. Approximately can mean that the standard deviation from one centred wavelength to the adjacent equally spaced centred wavelength accounts for less than 20%. In the present non-limiting example, surface-mounted LEDs 2 with the following spectral characteristics were selected:

| LED choice | Central wavelength | Half width |
|---|---|---|
| 1 | 420 nm | 13 nm |
| 2 | 450 nm | 20 nm |
| 3 | 470 nm | 20 nm |
| 4 | 520 nm | 30 nm |
| 5 | 590 nm | 14 nm |
| 6 | 630 nm | 15 nm |
| 7 | 810 nm | 30 nm |

Each LED 2 holds a maximum power rating of 200 mW.

The table also contains a seventh, not illustrated LED, which is used in one example which will be explained in the following.

Some of the spectral bands selected, for instance, those for LED 2 and 3 mentioned in the table before, can be selected to overlap at least partially. Others, for example, LEDs 4 and 5 are chosen to non-overlap.

In the context of the present disclosure, the sources of the table above may be referred to as "primary sources" or "light-emitting members" with equivalent meaning. All primary sources 1-6 are situated in the visible part of the spectrum, while source 7 is in the near infrared spectrum. In an alternative embodiment, source 7 may have a central wavelength λ = 830 nm.

A LED driver circuit (not illustrated) is also placed on the same PCB 3 and operably connected to each LED 2. The LED driver circuit supplies each LED 2 with an individual supply current. The LED driver controls the amperage of each supply current circuit selectively. Preferably, the LED driver circuit controls the amperage of each supply current using a pulse-width modulation technique in which the duty cycle of each pulse contained in the supply current is controlled. Adjusting the duty cycle allows the brightness (luminous flux) to be controlled and adjusted. The frequency in which the supply current is pulsed is preferably higher than 100 kHz, which results in a non-flicker emission of light for human eye. Alternatively, the LEDs 2 can be supplied with an adjustable DC current, outputted by the LED driver circuit to each LED 2, which results in no flickering at all. This can be useful when light pulses, regardless of the frequency, would make imaging, assessment, or treatment impossible. Using high frequency current or DC current is especially advantageous for the silent substitution, as the stimuli of the receptors remain constant.

A microcontroller (not illustrated), acting as a central control instance for the light source 1, is also placed on the PCB 3. The microcontroller is operably connected to the LED driver circuit, whereby the LED driver circuit sets and controls the current supplied to the LEDs 2 in response to control commands sent by the microcontroller to the LED driver circuit. The microcontroller mentioned is configured to selectively energise the LEDs 2 by instructing the LED driver circuit so that only one LED 2 of the plurality of LEDs 2 emits light. The microcontroller is further provided by means of the LED driver circuit to energise the plurality of LEDs 2 in consecutive order, meaning that one LED 2 after the other is energised, whereby two, three or any number thereof may be energised simultaneously. In particular, the microcontroller is configured to simultaneously energise any combination of LEDs 2 (including all LEDs 2). The microcontroller is also arranged to control the luminous intensity of each LED 2 by instructing the LED driver circuit to control the supply current correspondingly, such that all LEDs 2 emit light with the same luminous intensity when energised. This can result in an even spectral power distribution, which is highly desirable when emitting homogeneous light. Alternatively, or in addition, the microcontroller is also arranged to control the luminous intensity of each LED 2 individually by instructing the LED driver circuit to control the supply current correspondingly, such that a corresponding LED 2 emits light with a predetermined luminous intensity when energised.

The PCB 3 is arranged in thermal contact with a plurality of Peltier elements 4. Each Peltier element 4 is preferably arranged so that it is located below an LED 2, whereby the PCB 3 separates the Peltier element 4 from the corresponding LED 2. When energised, each Peltier element 4 creates a temperature gradient whereby the surface of the Peltier element 4 facing the LED 2 cools and provides refrigeration through a thermal path over the PCB 3, and the surface facing away from the LED 2 heats. The heating surface is thermally connected to a heat sink 5 that dissipates the generated heat to the environment. The function of the Peltier elements 4 is to keep the LED chip temperature constant during operation to ensure a constant luminous intensity during operation. One temperature sensor operably connected to the microcontroller can be placed directly adjacent to one LED 2. The temperature signal supplied by the temperature sensor can be used by the microcontroller to control the supply current supplied to the Peltier elements 4. The temperature gradient mentioned before can be controlled by the microcontroller. Supplying more current to the Peltier elements 4 results in a higher temperature gradient and finally in higher refrigeration. The temperature sensor mentioned can also be used to terminate the light emission by the LEDs 2, if the temperature determined with the temperature sensor is higher than a predetermined threshold, e.g. 50 °C. The microcontroller correspondingly instructs the LED driver circuit to stop supplying current to the LEDs 2.

The light emitted by each LED 2 is decoupled from each LED 2 by means of an optical fibre 6 assigned to each LED 2. The optical fibre 6 is optically coupled to the respective LED 2. In particular, it is important that the optical connection end of the optical fibre 6 is as close as possible to the LED chip to achieve a sufficient optical coupling with low losses. Alternatively, or in addition, an optical system, e.g. with lenses, can be used to collect the light of each LED and inject it into the corresponding optical fibre 6. However, this comes at additional costs. Each optical fibre 6 is configured as multi-mode fibre with a core made of glass or plastic material. Multi-mode fibres were selected because they allow an easier optical connection compared to single-mode fibres due to the larger core diameter. In addition, they can conduct light containing a wide range of wavelengths and allow an extended acceptance angle at the optical connection point, which also improves light coupling.

The fibre's distal ends with respect to the LEDs' optical connections, are mechanically fixed to form a bundle, whereby the shape depends on the subsequent optics (which will be explained below in greater detail). The surfaces of the optical cable ends can be polished to reduce or eliminate intensity losses due to scattering. Even though only six optical fibres 6 and 6 corresponding LEDs 2 are depicted in Fig. 1a, a different number of elements can be foreseen. In particular one further fibre 6 can be foreseen alongside the six illustrated fibres 6. The seventh optical fibre can also be integrated into the fibre bundle, preferably directly in the centre, whereby the other six fibres 6 are arranged to surround the seventh optical fibre. The seventh fibre may be optically connected to an external light source (external to the PCB 3) that can be operated independently of the LEDs 2 of the light source 1. The external light source may be, among others, a laser light source capable of emitting light in a narrow spectral band with high radiant intensity. The central wavelength of the external light source may be between 800 nm and 830 nm, preferably at 810 nm or 820 nm. This wavelength is preferably used in the treatment of eye diseases. Alternatively, in addition to the six previously mentioned LEDs 2, a seventh LED can be foreseen on the PCB that emits light with the previously mentioned central wavelength. The light from the seventh LED is injected into the seventh aforementioned optical fibre (instead of using an external light source). The microcontroller in concert with the LED driver circuit control and supply the seventh LED with energy. If the light source 1 is used, for example, in the diagnostic and treatment of eye diseases, a light mixture of the six mentioned LEDs 2 can be used to illuminate the fundus, whereby the seventh LED can be used to emit light with high energy of said spectral band or wavelength onto the retina to stimulate retinal cells at a specific point. The microcontroller can therefore control the LED driver in a first operating mode, in which the light source 1 is used to illuminate the fundus exclusively, and in a second operating mode, the light source is used to stimulate retinal cells utilizing the emission of the seventh LED. If an external light source for the purpose of stimulation is used, the microcontroller might only control the operation of the six LEDs, as outlined before.

The formed fibre bundle can be optically connected to the absorptive filter, which can be configured as a grey filter 7. More preferably, the grey filter 7 can be placed at the optical output, such that the mixed light passes the grey filter 7. The grey filter 7 is used for low light intensity excitation or stimulation, which can increase the dynamic range of the light source 1, especially when combined with subsequent optics. However, the grey filter is optional, and the formed fibre bundle can be directly optically connected to the optical light mixing member 8. Said optical light mixing member 8 collects the light at its optical input emitted by each fibre 6, and mixes, thereby creating a uniform (spectral and intensity) light distribution along the entire cross-section of the optical light mixing member 8. The detailed configuration of the optical light mixing member 8 will be explained in Fig 2 in greater detail. A light-collecting fibre tip polished at 45° related to the horizontal axis of the optical light mixing member 8 may collect and transmit the light from the optical output. The diameter of the light-collecting fibre tip must be smaller to the diameter of the optical light mixing member 8. The use of the light-collecting fibre tip is optional. However, when used, the light outputted at the optical output can be decoupled from said output using the light-collecting fibre tip and guided to a measurement device, such as a spectroscope, external to the light source 1. The optical output of the optical light mixing member 8 (in the absence of the light-collecting fibre tip) is already sufficient for emitting uniform and spectral homogeneous light. Alternatively, or in addition, the arrangement as illustrated in Fig. 1b is coupled to the said optical output (with or without the light-collecting fibre tip). It needs to be noted that the light exiting the optical output is light with a uniform light distribution, acting as a Lambert source with a cosine profile. However, this may depend on the shape of the light mixing member 8.

The optical output can be combined with a diffuser 9, as illustrated in Fig. 1b. This can further diffuse or scatter light emitted at the optical output, which can lead to a further improvement in uniform illumination. Preferably a diffractive diffuser, such as those made of fused silica, is used. The diffuser 9 can also be combined with the light-collecting fibre tip 10, whereby the light-collecting fibre tip 10 is directly optically connected to the optical output. The aperture stop 11 is placed in front of the diffuser 9 and is used to limit the amount of light emitted from the optical output. In the application as envisaged, it is used to imagine the pupils' eye in a Maxwellian arrangement so that the image is small enough to enter the eye without interfering with the iris. But for other applications, such as standard illumination applications, the aperture stop 11 is of no real use because the light intensity can be adjusted electronically, e.g. by controlling the supply current, as mentioned before. Finally, a shutter is used, such that any light can be prevented from leaving the optical output of the optical light mixing member 8.

As indicated before, Fig. 2 illustrates variants of the optical light mixing member 8a-8c, which may be selected or used depending on the intended application of the light source. The optical light mixing members 8a-8c use the total internal reflection to homogenise the light from the inhomogeneous light exiting the fibre bundle regardless of the spectral properties of the light outputted. Compared to other optical elements for mixing light, such as an integrating sphere, all optical light mixing members 8a-8c disclosed in the following offer the advantage of low optical losses.

The optical light mixing member 8a, shown at the top, is configured as an elongated straight glass rod with a hexagonal shape. The material used is fused silica, whereby the outer surface of the optical light mixing member 8a shown preferably is uncoated, but a sheathing can be used to prevent environmental radiation. The numerical aperture of the optical light mixing member 8a is close to 1, whereby the long diagonal accounts for 3 mm and the length accounts for 40 mm.

The optical light mixing member 8b, shown in the centre, is configured as an elongated optical fibre, preferably made of optical glass, thereby providing a good light conductivity with low losses. The optical fibre is uncoated, but a sheathing can be used to prevent environmental radiation. Under some circumstances, a coating would be better to reduce the reacting losses at the input and to reduce the numerical aperture of the output. The core diameter is constant over the entire horizontal extension and accounts for 3 mm. The length accounts for 40 mm.

The optical light mixing member 8c, shown at the bottom, is as configured as the optical light mixing member 8b shown in the centre, whereby the shape differs in that the elongated body is tapered along the horizontal extension. Preferably also optical glass is used, whereby a plastic material can be used instead. Plastic material is cheaper but might come at the cost of higher optical losses, especially for light with a wavelength shorter than that of visible light. The tapered optical fibre can be manufactured by using the optical light mixing member 8b shown in the centre, heating in the middle section and applying a pulling force to one end of the elongated optical fibre while holding the opposite end. The non-tapered end is configured with a diameter of 3 mm, and the tapered end is provided with a diameter of 1 mm. The tapered fibre has a length of 60 mm. The tapered fibre also offers the advantage that the light is condensed on the way to the tapered end, and the radiance at the optical output is increased as the surface area is decreased.

Fig. 3a illustrates a retinal diagnostic and/or therapy device 30 with the light source 1 as explained in Fig. 1a, in particular in the arrangement with the optical light mixing member configured as tapered fibre and with the aperture stop (see Fig. 1b) placed in series to the optical output. Of course, this is a very simplified representation because features such as evaluation optics that enable the clinician to take accurate measurements are not depicted but must be thought of by a technical person.

The optical arrangement is based on the Maxwellian view in which the exit pupil of the optical output is projected on the eye's entrance pupil 20. The light exiting the optical output of the light source 1 is imaged through the lens 12 to the pupil 21 of the eye 20, meaning that the optical output and the pupil 21 are conjugate. The rays continue illuminating the fundus 22, whereby plain lines exiting the optical output of the light source 1 represent two marginal rays. The field stop 11 conjugates the fundus 22 and limits the field of view to exceed 100°. In the present example the field of view can account to 60°. The dashed lines represent the optical rays coming form the field stop 11 and forming an image on the retina (in that case only the central object is represented). This field stop 11 can be configured differently, depending on the application, such as an obscuration in the centre. It needs to be noted that the present example makes use of the setup of the light source 1 comprising the six LEDs. Suppose a seventh LED (or an external light source as explained before) is used. Alternatively, or in addition, two retinal diagnostic and/or therapy devices of the same configuration may be provided, operating in parallel to allow simultaneous diagnosis and/or treatment of both eyes.

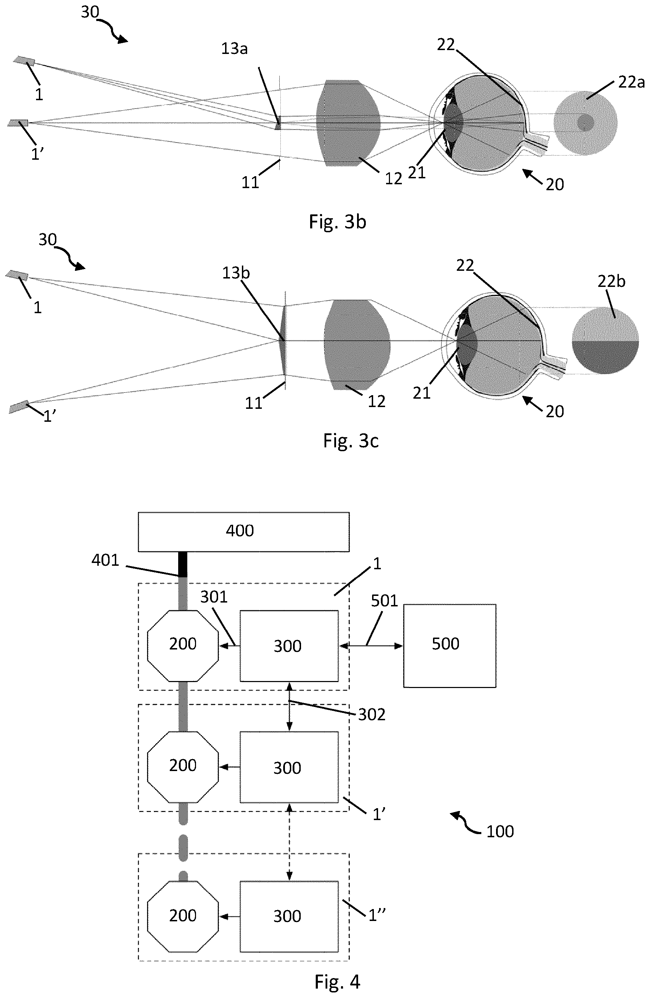

Fig. 3b shows a variant of a retinal diagnostic and/or therapy device of Fig. 3a. Two light sources 1, 1' are arranged alongside, whereby the optical outputs are spaced apart. Light exiting the two optical outputs is coaxially collimated with each other using the wedge prism 13a as part of the field stop 11 and collected by the lens 12. Two marginal rays are passing the pupil 21. As it can be noticed in the figure two different fields of light 22a are projected to the fundus 22. The inner circle corresponds to the stimulation field; the outer ring refers to the background field for illuminating the fundus 22. The first light source 1 can be configured in the configuration with six LEDs. The second light source 1' can comprise the seventh fibre connected to the external source emitting to stimulate the retinal cells. The optical setup illustrated is at the same time that simple, effective, and complex optics with a multiplicity of lenses can be eliminated.

Fig. 3c shows a further variant of a retinal diagnostic and/or therapy device of Fig. 3a. Two light sources 1, 1' are also arranged alongside, whereby the optical outputs are spaced apart. Light exiting the two optical outputs is collimated with each other using the roof prism 13b placed in the field stop 11 and collected by the lens 12. Two chief rays are passing the pupil 21. As it can be noticed in the figure, two directly adjacent fields of light 22b are projected to the fundus 22. The two light sources 1, 1' can be configured in the configuration with six LED, whereby they are controlled to emit light at the optical outputs with a mutually different spectrum or radiant flux. The advantage is that with the example optical setup, it is possible to stimulate the two fields differently, for example, to determine the precise photoreceptors sensitivity or simply to stimulate one field while the other remains constant.

**Fig. 4** schematically illustrates a system of light sources 100, each light source 1, 1', 1" being configured as shown in Fig. 1a, meaning that each light source 1, 1', 1" is configured with an optical output as explained before. The light exiting each output can be collected by and combined in subsequent optics such that a polychromatic spectral homogeneous light is formed. The component collecting the light can be a prism or a lens, such as those explained hereinbefore. Alternatively, there could also be no component collecting the light, but each light source 1, 1', 1" is added to an illuminated object or to provide a very large spectrally homogeneous field for experiment with mice to give them a spectrally homogeneous light environment. Even though this is an important aspect of how the light emitted by the plurality of output is collected and combined, the explanation will be focused on the control of the system of light sources 100.

Each light source 1, 1', 1" comprises the PCB, the LEDs (six or seven), the LED driver circuit for driving the LEDs. The three components mentioned are named in the following as light engine 200. The microcontroller is operably connected to the LED driver circuit trough the control bus 301 as explained hereinbefore. Each light source 1, 1', 1" also comprises at least one temperature sensor and the Peltier elements. Alternatively, the Peltier elements are omitted, and the PCB carrying the LEDs is passively cooled using the heat sink. Even though only three light sources 1, 1', 1" are illustrated, the system of light sources 100 can feature any number of equally or differently configurated light sources depending on the application and the total radiant flux to be emitted.

Through the master communication bus 501 the microcontroller 300 of the first light source 1 receives a protocol from the master control unit 500, configured as a computer device. The protocol defines, among other things, the chronological order and the radiant flux with which the LEDs of the first light source 1 are to be operated. The protocol can either be looped through to the microcontrollers 300 of the other light sources 1', 1" or the microcontroller 300 of the first light source 1 sends an adapted protocol to the microcontrollers of the other light sources 1', 1" to synchronise the sequence of emission with the first light source 1. The protocol is looped or sent to the other microcontrollers 300 through the communication bus 302. Alternatively, or in addition, the communication bus 302 can be used to synchronise the light sources 1, 1', 1" (=the emission) with each other. Each microcontroller 300 translates the protocol received into a multi-bit PWM signal, preferably a 12-bit PWM signal, which is supplied to the LED driver circuits via the control bus 301. The LED driver circuit of the individual light engines 200 sets the radiant flux of each LED comprised in the light engine 200 according to the protocol. The electrical power is supplied by a common power supply 400, whereby each light source 1, 1', 1" is connected to the supply bus.

The master control unit 500 can send, in addition to the protocol, start and stop commands to the microcontroller 300 of the first light source 1. The protocol is executed from the start command on each microcontroller and can be interrupted by the stop command; otherwise the protocol is executed till the end. During the execution of the protocol, information is sent to the master control unit 500 via the master communication bus 501 to inform the state of the protocol and further information, such as the temperature measured by the at least one temperature sensor. The information can also comprise further measured values, such as the current supplied by the LED diver circuits to the LEDs.

In embodiments, the inventive source is applied to a combined binocular silenced pupillometer equipped with infrared cameras that allow simultaneous and bilateral monitoring and measuring of the pupils. A pair of the inventive sources is used to provide a bilateral, independently settable silent substitution stimulation of a subject's eye fundus.

As mentioned already, the binocular pupillometer of this embodiment uses a Maxwellian optic arrangement. The exit pupil of the light sources is designed to be smaller than the smallest eye pupil that can statistically be expected. The light is not vignetted by the iris and the luminous flux incident on the retina is not altered by changes in eye pupil diameter.

The pupillometer integrates a plurality of cameras for the automatic determination of the position of the subject's pupil, the gaze direction and the pupil's diameter. Preferably, the cameras use an independent IR illumination system that does not interfere with the optical stimulation. In a preferred arrangement, at least one eye is monitored simultaneously by two spaced-apart cameras, whereby the position of the pupil can be determined in real time through triangulation of the images taken by the two cameras. This information allows a precise alignment of the source exit pupil with the eye. In a variant, one camera is positioned on the side of one eye, while the other is mounted below the target eye.

Preferably, the cameras can be set to align the plane of focus with a frontal plane of the eye using the Scheimpflug principle.

The pupillometric information is extracted by an automatic image processing system that determines, through suitable image processing algorithms, the ellipse that most closely approximates the pupil's edge. It is enough to apply this analysis to the images of one of the cameras, for example the side-mounted one. The contralateral eye may be imaged by a single side-mounted camera that extracts, in a similar way, the corresponding pupillometric information.

As already mentioned, the light sources of the invention have a predefined number of primary sources-six or seven LEDs in the embodiment disclosed above-having diverse wavelengths. The inventors have determined that certain combinations of primary wavelengths are especially effective in generating silencing stimuli for the photoreceptors of human retina. In particular, LED 1, 2, 3 have their central wavelengths at 420 nm, 450 nm, 470 nm in the violet/blue region of the spectrum. This arrangement enables the generation of high-contrast silencing signals for the S-cones and the melanopsin-containing retinal ganglion cells (mRGCs) whose response overlaps significantly in the blue. Another important feature is the choice of one primary source (LED 5) at about 590 nm, in the yellow part of the spectrum. Thanks to this combination comprising one or more blue/violet primary sources, at least one primary source in each of the yellow, green and red spectral colours, the light source can deliver Michelson contrast for mRGCs higher than 50%, preferably higher than 75%, more preferably higher than 80%. Michelson contrast is defined as the ratio *m* = (*Iₘₐₓ* - *Iₘᵢₙ*) / (*Iₘₐₓ* + *Iₘᵢₙ*). Another feature contributing to a favourable Michelson contrast is the selection of single-wavelength LEDs as primary sources, with bandwidth smaller than 30 nm, rather than phosphor-based LEDs.

The embodiments above disclose a thermal management system with temperature sensors and Peltier elements or other forms of active cooling for the stability and consistency of the primary LED sources. In combination or in alternative, the light source of the invention may also have temperature-compensated LED drivers, that change the driving current based on a temperature measurement in order to maintain the luminosity of the primary sources at the nominal level, or optical-feedback correction systems, in which the LED drivers can correct the driving current based on a signal from a photodetector, for the same purpose.

In another embodiment, not represented, the light mixing member may be realised by providing a bundle of optical fibres per each primary source, to collect the generated light, and then combining the bundles coming from the primary sources into a thicker bundle of fibres in which the individual fibres are shuffled and intertwined randomly.

### Reference symbols in the figures

- 1: Light source, first light source
- 1': Second light source
- 2: Light-emitting diode (LED), LEDs
- 3: Printed-circuit board (PCB)
- 4: Peltier elements
- 5: Heatsink
- 6: Optical fibres
- 7: Absorptive filter, grey filter
- 8: Optical light mixing member
- 8a: Hexagonal light pipe, glass, straight
- 8b: Optical fibre, glass
- 8c: Tapered optical fibre
- 9: Diffuser, optical light mixing member tip
- 10: Fibre tip
- 11: Aperture stop, field stop
- 12: Lens
- 13a, 13b: Prism
- 20: Eye
- 21: Pupil
- 22: Fundus
- 22a, 22b: Light pattern/fields on the retina
- 30: Retinal diagnostic and/or therapy device
- 100: System of light sources
- 200: Light engines
- 300: Control unit, microcontroller
- 301: Control bus
- 302: Communication bus
- 400: Power supply
- 401: Supply bus
- 500: Master control unit, computer device
- 501: Master communication bus

## Claims

1. A light source (1) for emitting polychromatic spectral homogeneous light in a spectral band, the light source (1) comprising
- a plurality of light-emitting members (2), wherein each light-emitting member (2) of the plurality of light-emitting members (2) is configured to emit light in a specific single spectral band, the said single spectral band being **characterised by** a central wavelength and a spectral bandwidth; and
- a number of light guides (6) corresponding to the number of light-emitting members (2), each light guide (6) being optically connected at one end to a corresponding light-emitting member (2), the plurality of light guides (6) being arranged at another end to form a light guide bundle; and
- an optical light mixing member (8), the optical light mixing member (8) is configured with an optical input and an optical output, wherein the optical input is optically connected to the light guide bundle, and wherein the optical light mixing member (8) is configured to mix and homogenise spectrally the light emitted by the plurality of light-emitting members (2), wherein the optical output is provided to output the mixed and homogenised light for emitting the polychromatic spectral homogeneous light.

2. The light source (1) of claim 1, wherein the light-emitting members are light emitting diodes (LEDs) (2), each having a central wavelength between 400 nm and 700 nm.

3. The light source (1) of any one of the preceding claims, comprising a light-emitting member (2) in excess of the number of a plurality of light-emitting members (2), wherein said excess light-emitting member (2) is configured to emit light in a mutually different spectral band, wherein the mutually different spectral band comprises or is centred at a wavelength between 790 nm and 830 nm, or 800 nm and 820 nm or at 810 nm.

4. The light source (1) of any one of the preceding claims, wherein the light mixing member (8) is one of: a straight hexagonal glass light pipe (8a), preferably configured with a long diagonal of 3 mm and a length of at least 40 mm, an optical fibre with a constant diameter (8b), preferably configured with a diameter of 3 mm and a length of at least 40 mm, or a tapered optical fibre (8c), wherein a non-tapered end corresponds to the optical input of the light mixing member (8), and preferably the non-tapered end is configured with a diameter of 3 mm and a tapered end is configured with a diameter of 1 mm.

5. The light source (1) of any one of the preceding claims, wherein the light guide bundle is formed in a shape corresponding to a shape of the light mixing member (8), and wherein a surface area of the said shape equals a surface area of the optical input of the optical light mixing member (8).

6. The light source (1) of any one of the preceding claims, further comprising an absorptive filter (7) for excitation of the light emitted by the plurality of light-emitting members (2), wherein the absorptive filter (7) is in optical connection with the optical input of the optical light mixing member (8) and the light guide bundle.

7. The light source (1) of any one of the preceding claims with three light emitting members having different central wavelengths in the blue/violet part of the spectrum.

8. The light source of any one of the preceding claims, having a light emitting member with a central wavelength in the yellow part of the spectrum.

9. The light source (1) of claim 2, further comprising a LED driver circuit operably coupled to the plurality of LEDs (2), configured to selectively drive each LED (2) from the plurality of LEDs (2).

10. A system of light sources (100), comprising a plurality of light sources (1) of any one of the preceding claims;
- a master control unit (500) operably coupled to each control unit of the plurality of light sources (1), wherein the master control unit (500) is configured to control the radiant flux of each light-emitting member (2) comprised in the plurality of light sources (1) as a function of time, by providing control signals to each control unit.

11. The system of light sources (100) of the preceding claim, comprising a light collecting device with a collector input and a collector output, wherein each optical output of the plurality of light sources (1) is optically connected to the collector input, wherein the collector output is configured to output light with a radiant flux corresponding to approximately a sum of radiant fluxes received at the collector input.

12. A device (30) comprising:
- at least one light source (1) of any one of the claims 1 to 9;
- a lens (12) optically connected to the optical output of the at least one light source (1), wherein the lens (12) is configured to collect the polychromatic spectral homogeneous light outputted by the light source (1) and is further configured to send the light collected onto a retina (22) of an eye (20) external to the device (30).

13. The device (30) of the preceding claim, further configured to emit two different fields of light to the eye or eyes (20), comprising a first and a second light source (1, 1') of any one of the claims 1 to 9, each optical output is optically connected to the lens (12), wherein a first field of light is output by the first light source (1) and a second field of light is output by the second light source (1').

14. The device (30) of claim 14, further comprising a refracting or reflecting element (13a, 13b) in optical connection with the optical outputs of the first and the second light source (1, 1'), wherein said element (13a, 13b) is configured to optically align the polychromatic spectral homogeneous light emitted by each light source (1, 1').
